# EUROPEAN PATENT APPLICATION

(11) **EP 4 686 449 A1**
(43) Date of publication of application: **04.02.2026**
(21) Application number: 24199182.7
(22) Date of filing: 09.09.2024
(51) Int. Cl.: A61B 8/00, G01S 7/00, G01S 7/52

(54) **ULTRASOUND SYSTEM**

(30) Priority: 31.07.2024 US 202463677629 P
(71) Applicant: Koninklijke Philips N.V., 5656 AG Eindhoven (NL)
(72) Inventor: SHERMAN, Jeffrey, Eindhoven (NL); ROBINSON, Andrew Lee, Eindhoven (NL)
(74) Representative: Philips Intellectual Property & Standards

(57) **Abstract**

The invention relates to data transmission within an ultrasound system, wherein the ultrasound system comprises a host (130), the host comprising a host connector (132); an ultrasound probe; and a cable (120) connected to the ultrasound probe, the cable comprising: a cable connector (122), wherein the cable connector is configured to connect with the host connector, and wherein the cable connector comprises a high-speed transmitter to improve data transmission while maintaining acceptable power and thermal budgets in the ultrasound probe handle.

## Description

### FIELD OF THE INVENTION

The invention relates to ultrasound systems, and in particular, to digital ultrasound systems, comprising a probe, a host, and a cable between the probe and the host. The invention also relates to a cable connector, a cable, and a probe for use in such ultrasound systems, and to a method for transmitting ultrasound data.

### BACKGROUND OF THE INVENTION

Data transmission from an ultrasound probe to a host is a major bottleneck of most ultrasound systems. On the one hand as much data as possible is desired to be transmitted, leading to a large and bulky cable, while on the other hand a thin and light cable is desired for good ergonomics. In particular, sonographers often complain about hand, arm and shoulder fatigue, and stress that results from having to operate an ultrasound probe connected to a large and bulky cable.

Solutions to this problem could be going wireless or moving much of the data processing into the ultrasound probe. However, this comes at major limitations in terms of heat and power management. Ultrasound image quality degrades as probes get hotter, and when moving processing power into the probe, the electronic circuitry will inevitably consume more power, leading to higher temperatures in the probes.

### SUMMARY OF THE INVENTION

It is, inter alia, an object of the invention to provide improved data transmission between an ultrasound probe and an ultrasound system host. The invention is defined by the independent claims. Advantageous embodiments are defined in the dependent claims.

According to an aspect of the invention, there is provided an ultrasound system comprising:
a host, the host comprising a host connector;
an ultrasound probe; and
a cable connected to the ultrasound probe, the cable comprising:
   a cable connector, wherein the cable connector is configured to connect with the host connector, and wherein the cable connector comprises a high-speed transmitter.

In an example, the high-speed transmitter comprises an optical transmitter.

In an example, the cable connector and the host connector communicate via optical transmission. In an example, the high-speed transmitter comprises a serializer.

In an example, the cable comprises M data lanes, wherein *M* > 1,
the host connector comprises N data lanes, wherein *N* ≥ 1, and
the serializer is configured to serialize *M* data lanes to *N* data lanes, and wherein *N < M.*

In an example, the ultrasound probe further comprises an optical transmitter, and the cable is configured for optical transmission. In an example, the cable connector further comprises an optical receiver.

In an example, the high-speed transmitter in the cable connector is embedded on an Application-Specific Integrated Circuit (ASIC) or a Field-Programmable Gate Array (FPGA).

In an example, the ultrasound probe further comprises:
an analog to digital converter; and
a digital transmitter.

In an example, the digital transmitter comprises a plurality of low-speed transmitters that have a lower transmission speed than the high-speed transmitter in the cable connector.

According to another aspect of the invention, there is provided a cable connector comprising a high-speed transmitter.

According to another aspect of the invention, there is provided an ultrasound cable comprising a cable connector as defined herein.

According to another aspect of the invention, there is provided an ultrasound probe comprising a cable as defined herein.

According to another aspect of the invention, there is provided a method of transmitting ultrasound data, the method comprising:
generating ultrasound data responsive to:
   emitting ultrasound waves from a transducer array in an ultrasound probe; and
   receiving reflected ultrasound waves at the transducer array;
transmitting the ultrasound data through a cable connecting the ultrasound probe to a host, wherein the transmitting comprises increasing the transmission speed of the ultrasound data in a cable connector of the cable, wherein the cable connector connects to a host connector of the host.

These and other aspects of the invention will be apparent from and elucidated with reference to the embodiments described hereinafter.

### BRIEF DESCRIPTION OF THE DRAWINGS

Fig. 1 shows an exemplary ultrasound system.
Fig. 2 shows an exemplary cable connector and host connector.

### DESCRIPTION OF EMBODIMENTS

The invention relates to ultrasound. In particular to data or signal transfer between an ultrasound probe and a host of an ultrasound system. More particularly, to the arrangement of digital transmitters (e.g., low-speed transmitters and high-speed transmitters) throughout the ultrasound system.

Data and signal are used interchangeably throughout this specification.

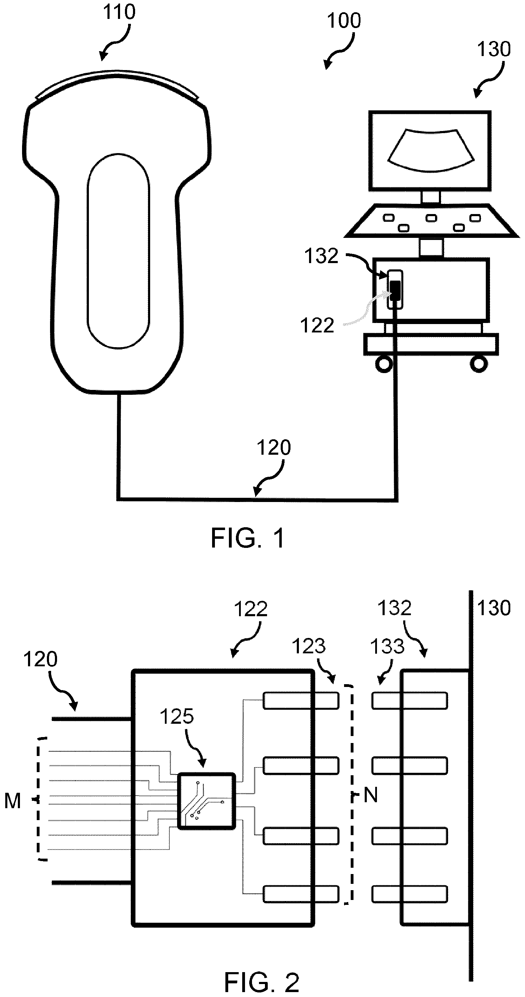

Fig. 1 shows an example ultrasound system 100 comprising a probe (handle) 110 and a host 130 connected via a cable 120. A detailed description of an ultrasound system comprising transducer array, beamformers, controllers, analog to digital converters, signal processors, etc. is considered known by the skilled person and will not be further discussed.

In the example of Fig. 1, the cable 120 is permanently connected to the probe 110 while being removably connected to the host via a cable connector 122 that connects with a host connector 132. In other examples, the cable 120 may also be removably attached to the ultrasound probe 110. The ultrasound system 100 is preferably a digital ultrasound system wherein the ultrasound probe may further comprise an analog to digital converter. The ultrasound probe may further comprise one or more digital transmitters (e.g., low-speed transmitter or high-speed transmitter, wherein a low-speed transmitter is configured to transmit at a lower speed than the high-speed transmitter).

When the cable 120 is connected to the host 130 via the cable connector 122 and the host connector 132, connection pins of the cable connector 122 and host connector 132 are put in contact with each other to enable a transfer of signals from the cable to the host via the connector pins. Fig. 2 shows an exemplary cable connector 122 comprising 4 connector pins 123 and an exemplary host connector 132 comprising 4 pins 133. In Fig. 2, the connectors 122 and 132 are positioned opposite to each other but not yet connected. Connection is made upon contact between connector pins 123 and 133.

In a preferred embodiment, an ultrasound system thus comprises a host 130 with a host connector 132, an ultrasound probe 110, and a cable 120 connected to the ultrasound probe. The cable further comprises a cable connector 122 configured to connect with the host connector and the cable connector further comprises a high-speed transmitter 125. The ultrasound probe 110 may be an external ultrasound probe, e.g., a general imaging ultrasound probe, a cardiac ultrasound probe, and the like, or an internal ultrasound probe, e.g., a transthoracic ultrasound probe, a transesophageal ultrasound probe, an intervaginal ultrasound probe, and the like. The ultrasound probe may be an ultrasound imaging probe and the ultrasound system may be an ultrasound imaging system.

The high-speed transmitter 125 is in particular configured to increase the transmission speed of signals arriving via the cable 120 before transmitting these signals via the connector pins 123 into the host 130 via connector pins 133. For example, signals arriving via the cable 120 may be transmitted at a speed of 2 Gb/s (Giga bits per second), and the high-speed transmitter may increase the transmission speed to 4 Gb/s. It should be obvious to a skilled person in the art that any factor in transmission speed increase (e.g., 2 (from 2 Gb/s to 4 Gb/s), 3 (from 2 Gb/s to 6 Gb/s), 4 (from 2 Gb/s to 8 Gb/s), 5, 6, 8, 9, 10, 11, 12, 13, 14, ...) is possible as long as the high-speed transmitter supports said increase. A suitable high-speed transmitter is considered known to a skilled person in the art. In an example, the high-speed transmitter in the cable connector may be embedded on an Application-Specific Integrated Circuit (ASIC) or a Field-Programmable Gate Array (FPGA).

A high-speed transmitter is often desirable to reduce complexity, but it may be impractical to put into the probe handle given power and thermal constraints. Placing the high-speed transmitter into the connector allows the heat to dissipate away from the patient and sonographer in a safer region.

Having the high-speed transmitter as depicted in Fig. 2 in the cable connector thus has the advantage that no, or a less powerful high-speed transmitter need to be placed in the ultrasound probe 110. In fact, ultrasound probes often comprise high-speed transmitters. However, due to power and thermal issues the in-probe high-speed transmitters are often limited in their ability to speed up transmission. More powerful high-speed transmitters for example consume more power which is not always available in the ultrasound probe. Furthermore, even if additional power were available, with a higher power consumption comes a higher thermal output. In other words, more powerful in-probe high-speed transmitters may heat up the ultrasound probe beyond an acceptable temperature for the ultrasound transducer array, thereby negatively influencing image quality. The inventors have found that for example, placing multiple low-speed transmitters in the probe 110 may be more energy and thermally efficient than a high-speed transmitter in the probe. However, in order to still be able to connect to a host system, it is preferable to then place a higher-speed transmitter in the cable connector. The low-speed transmitters would thus be digital transmitters with a lower transmission speed than the high-speed transmitter placed in the cable connector. By placing the high-speed transmitter or an additional high-speed transmitter in the cable connector, the power consumption and heat creation are moved away from the probe. It is unlikely that the heat generated by the high-speed transmitter in the cable connector would travel through the cable to the ultrasound probe. Particularly considering the large surface area of the cable which would dissipate all the heat generated at the cable connector before reaching the probe. Furthermore, it would be easy to modify a host connector to supply additional power to the cable connector only to power the high-speed transmitter.

Additionally, placing a high-speed transmitter in the cable connector allows for reducing the number of connector pins in the cable and host connectors. By reducing the number of connector pins, the complexity of the connectors may be reduced. Furthermore, reducing the number of connector pins can beneficially influence a reduction of faulty connections between the cable connector and the host connector. For example, it has been observed that dirt inside connectors can disrupt data transmission from a cable connector pin to a host connector pin. By reducing the number of pins a smaller connector may be designed which is more resistant against dirt insertion. Such connectivity issues between connector pins have also been observed after water insertion into either the cable or the host connector.

Additionally, placing a high-speed transmitter in the cable connector allows for different designs of the ultrasound system. For example, the lane count through the cable may be increased while still being able to use an old host. Thereby newer probes with higher lane counts can be made compatible with older hosts that comprise lower lane counts at their respective host connectors. This is particularly advantageous with the rise in digital ultrasound probes, which often require many more data or signal lanes than more conventional analog ultrasound probes. According to this invention, a digital ultrasound probe can thus be made compatible with an older host system.

For example, an exemplary digital ultrasound probe may comprise 128 channels sampled at 20 MS/s (Mega-Samples per second) with 10-bit resolution which would require a transmission speed of 25.6 Gb/s (Gigabit per second) before encoding (128 × 20 × 10⁶ × 10 = 25.6 × 10⁹). 8 lanes at 4 Gb/s, for a total data capacity of 32 Gb/s would provide sufficient capacity. However, the lane speed of 4 Gb/s may not always achievable from the ultrasound probe due to constraints in the power and thermal budgets as described above, or in the capacity of a single lane; for example, it may be necessary to limit the lane speed to 2 Gb/s. However, an old host may be designed with an interface to support 8 lanes. Therefore, there exists a desire to be able to transmit at more lanes, while still being able to use host system connectors with a reduced lane count.

According to an example, the high-speed transmitter is a serializer. A serializer is in general configured to serialize signals coming from *M* data lanes into *N* data lanes, wherein *M* is greater than *N*. For example, 2 signals received by the serializer in parallel through two different data lanes can be configured by the serializer to be transmitted in sequence through a single data lane. Advantageously, the 2 parallel signals received were at a first speed, e.g., 2 Gb/s, and the single signal sent by the serializer transmits the 2 signals in sequence at 4 Gb/s. In that way, there is no reduction in overall transmission speed as the same information is still transmitted in the same amount of time. In the particular example shown in Fig. 2, the serializer serializes data from 16 data lanes (*M*) at rate *R*1 to 8 data lanes (*N*) at rate *R*2 where *R*2 > *R*1. Each line and connector pin as drawn in Fig. 2 represents a data lane or lane. In an example, the cable comprises *M* data lanes, wherein *M >* 1, the host connector comprises *N* data lanes, wherein *N* ≥ 1, and the serializer is configured to serialize *M* data lanes to *N* data lanes, and wherein *N < M. M* and *N* are in general positive integer numbers. In a preferred embodiment *M* = *2N.*

According to an example, the high-speed transmitter is an optical transmitter configured to convert electrical signals to optical signals. The data rate would further be increased by the optical transmitter and the system could exploit the generally higher transmission rate in optical transmission when compared to electrical transmission. In such cases, the cable connector and the host connector communicate via optical transmission. In particular, the cable connector pins and host connector pins may be configured for optical data transmission. The host or host connector may further comprise an optical receiver to convert optical signals back to electrical signals. In examples, the optical transmitter may also be placed in the ultrasound probe handle, and the cable could be configured for optical data transmission.

In examples, the ultrasound probe 110 may comprise an optical transmitter, the cable 120 may be configured for optical data transmission, and the cable connector 122 may comprise an optical receiver. The architecture would thus follow ultrasound probe comprising an optical transmitter -> optical transmission through cable -> optical receiver in cable connector -> high-speed transmitter in cable connector -> connection to the host connector and host. In this case, the cable connector and host connector would be configured for electrical data transmission which is more reliable for removable connectors than optical data transmission.

According to some examples, the data lanes or lanes may comprise copper wires, coaxial cables or fiber optic cables. The data lanes or lanes may be configured to transmit electrical signals or optical signals. The connector pins may comprise a conductive metal such as copper, brass, bronze etc. In this case, the connector pins may be configured for electrical signal transmission. The connector pins may comprise a metal or ceramic. In this case the connector pins may be configured for optical signal transmission.

A preferred embodiment comprises a cable connector comprising a high-speed transmitter.

A preferred embodiment comprises an ultrasound cable comprising a cable connector comprising a high-speed transmitter.

A preferred embodiment comprises an ultrasound probe comprising a cable comprising a cable connector comprising a high-speed transmitter.

According to an embodiment, a method of transmitting ultrasound data comprises
generating ultrasound data responsive to:
   emitting ultrasound waves from a transducer array in an ultrasound probe; and
   receiving reflected ultrasound waves at the transducer array; and
transmitting the ultrasound data through a cable connecting the ultrasound probe to a host, wherein the transmitting comprises increasing the transmission speed of the ultrasound data in a cable connector of the cable, wherein the cable connector connects to a host connector of the host.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the device claim enumerating several means, several of these means may be embodied by one and the same item of hardware. Measures recited in mutually different dependent claims may advantageously be used in combination.

## Claims

1. An ultrasound system (100) comprising:
a host (130), the host comprising a host connector (132);
an ultrasound probe (110); and
a cable (120) connected to the ultrasound probe, the cable comprising:
a cable connector (122), wherein the cable connector is configured to connect with the host connector, and wherein the cable connector comprises a high-speed transmitter (125).

2. The ultrasound system of claim 1,
wherein the high-speed transmitter comprises an optical transmitter.

3. The ultrasound system of claim 2, wherein the cable connector and the host connector communicate via optical transmission.

4. The ultrasound system of any one of the preceding claims, wherein the high-speed transmitter comprises a serializer.

5. The ultrasound system of claim 4,
wherein the cable comprises M data lanes, wherein *M* > 1,
wherein the host connector comprises N data lanes, wherein *N* ≥ 1, and
wherein the serializer is configured to serialize *M* data lanes to *N* data lanes, and wherein *N <M.*

6. The ultrasound system of claim 1, wherein the ultrasound probe further comprises an optical transmitter and wherein the cable is configured for optical transmission.

7. The ultrasound system of claim 6,
wherein the cable connector further comprises an optical receiver.

8. The ultrasound system of any of the preceding claims, wherein the high-speed transmitter in the cable connector is embedded on an Application-Specific Integrated Circuit (ASIC) or a Field-Programmable Gate Array (FPGA).

9. The ultrasound system of any of the preceding claims, wherein the ultrasound probe further comprises:
an analog to digital converter; and
a digital transmitter.

10. The ultrasound system of claim 9, wherein the digital transmitter comprises a plurality of low-speed transmitters that have a lower transmission speed than the high-speed transmitter in the cable connector.

11. A cable connector (122) comprising a high-speed transmitter (125).

12. An ultrasound cable (120) comprising the cable connector of claim 11.

13. An ultrasound probe (110) comprising the cable of claim 12.

14. A method of transmitting ultrasound data, the method comprising:
generating ultrasound data responsive to:
emitting ultrasound waves from a transducer array in an ultrasound probe; and
receiving reflected ultrasound waves at the transducer array;
transmitting the ultrasound data through a cable connecting the ultrasound probe to a host, wherein the transmitting comprises increasing the transmission speed of the ultrasound data in a cable connector of the cable, wherein the cable connector connects to a host connector of the host.
